# EUROPEAN PATENT APPLICATION

(11) **EP 4 035 670 A1**
(43) Date of publication of application: **03.08.2022**
(21) Application number: 20867960.5
(22) Date of filing: 25.09.2020
(51) Int. Cl.: A61K 35/28, A61K 48/00, A61P 1/02, A61P 1/16, A61P 3/10, A61P 9/10, A61P 11/00, A61P 11/04, A61P 13/12, A61P 17/02, A61P 19/02, A61P 19/08, A61P 25/00, A61P 25/28, A61P 27/02, A61P 35/00, A61P 37/02, A61P 43/00, C12N 1/00, C12N 5/0775, C12Q 1/6837

(54) **METHOD FOR MANUFACTURING REPAIRING AGENT FOR BIOLOGICAL TISSUE DAMAGE, AND REPAIRING AGENT FOR BIOLOGICAL TISSUE DAMAGE**

(30) Priority: 26.09.2019 JP 2019175648
(71) Applicant: Two Cells Co., Ltd, Hiroshima-shi, Hiroshima 732-0816 (JP); Hiroshima University, Higashi-Hiroshima-shi Hiroshima 739-8511 (JP)
(72) Inventor: KATO, Yukio, Hiroshima-shi, Hiroshima 732-0816 (JP); MAEDA, Satoshi, Hiroshima-shi, Hiroshima 732-0816 (JP); TSUJI, Koichiro, Hiroshima-shi, Hiroshima 732-0816 (JP); SHAO, Jin Chang, Hiroshima-shi, Hiroshima 732-0816 (JP); NAKASHIMA, Ayumu, Hiroshima-shi, Hiroshima 739-8511 (JP); MASAKI, Takao, Hiroshima-shi, Hiroshima 739-8511 (JP); DOI, Shigehiro, Hiroshima-shi, Hiroshima 739-8511 (JP); ISHIUCHI, Naoki, Hiroshima-shi, Hiroshima 739-8511 (JP)
(74) Representative: Plasseraud IP
(86) International application number: PCT/JP2020/036252
(87) International publication number: WO 2021/060460

(57) **Abstract**

The present invention provides a novel repair agent for damaged tissue that brings about a notably high effect of repairing damaged tissue, as compared with conventional repair agents for damaged tissue, and a method for producing such a repair agent. A method for producing a repair agent for damaged tissue of the present invention includes the step of culturing mesenchymal stem cells in a serum-free medium at an oxygen concentration of less than 5%.

## Description

### Technical Field

The present invention relates to a method for producing a repair agent for damaged tissue, and to the repair agent.

### Background Art

Mesenchymal stem cells (MSC) can be isolated not only from tissues such as bone marrow, adipose tissue, a synovial membrane, a dental pulp, and a periodontal membrane, but also from various tissues such as a placenta, cord blood, and an umbilical cord. In addition, the mesenchymal stem cells can be cultured and proliferated in vitro. Furthermore, the mesenchymal stem cells have multipotency that allows the mesenchymal stem cells to differentiate not only into mesenchymal cells (e.g., osteoblasts, adipose cells, and cartilage cells) but also into non-mesenchymal cells (e.g., neural precursor cells and hepatocytes). Thus, the mesenchymal stem cells are expected to be used as a material for production of cells for use in regenerative medicine or/and cell therapy.

The mesenchymal stem cells can be cultured by, for example, not only using a medium (serum medium) containing fetal bovine serum (FBS) but also using a serum-free medium in which fewer heterozoic proteins are mixed. For example, Patent Literatures 1 to 3 each describe a serum-free medium for use in culture of mesenchymal stem cells.

Mesenchymal stem cells have a wide variety of functions. Thus, the mesenchymal stem cells are expected to have many unknown functions. Under the circumstances, attempts have been made to find unknown functions of mesenchymal stem cells. For example, Patent Literature 4 discloses a repair agent for damaged tissue, containing mesenchymal stem cells cultured in a serum-free medium, and a method for producing the repair agent. According to the technique, mesenchymal stem cells are cultured at a normal oxygen concentration equivalent to the atmosphere, and the mesenchymal stem cells are used as an active ingredient of the repair agent.

### Citation List

### [Patent Literature]

[Patent Literature 1]
   International Publication No. WO 2007/080919
[Patent Literature 2]
   International Publication No. WO 2011/ 111787
[Patent Literature 3]
   International Publication No. WO 2015/016357
[Patent Literature 4]
   International Publication No. WO 2018/ 123968

### Summary of Invention

### Technical Problem

The repair agent for damaged tissue disclosed in Patent Literature 4 brings about a sufficient therapeutic effect but, if a repair agent for damaged tissue that brings about a higher therapeutic effect could be obtained, such a repair agent would be effective in the medical field.

Under the circumstances, an object of an aspect of the present invention is to provide a novel repair agent for damaged tissue that brings about a notably high effect of repairing damaged tissue, as compared with conventional repair agents for damaged tissue, and to provide a method for producing such a repair agent.

### Solution to Problem

In order to attain the object, a method of an aspect of the present invention for producing a repair agent for damaged tissue includes the step of: culturing mesenchymal stem cells in a serum-free medium at an oxygen concentration of less than 5%.

According to the method of an aspect of the present invention, the serum-free medium contains a fibroblast growth factor (FGF), a platelet derived growth factor (PDGF), an epidermal growth factor (EGF), at least one phospholipid, and at least one fatty acid.

According to the method of an aspect of the present invention, the mesenchymal stem cells are subcultured at least once in the culturing step.

In order to attain the object, a repair agent for damaged tissue in accordance with an aspect of the present invention contains mesenchymal stem cells in which a gene that encodes angiopoietin-like 4, a gene that encodes fatty acid binding protein 3, a gene that encodes delta-like 2 homolog (Drosophila), a gene that encodes fructose-bisphosphate aldolase C, a gene that encodes TRPM8 channel-associated factor 2, and a gene that encodes REST corepressor 2 are respectively expressed in amounts of at least 3 or more times, as compared with respective expression amounts thereof in mesenchymal stem cells which have been cultured in a serum-containing medium at an oxygen concentration of 5% or more.

The repair agent of an aspect of the present invention is intended to (i) suppress fibrosis of a living tissue, (ii) suppress infiltration of an inflammatory cell, or (iii) control activity of a macrophage.

For the repair agent of an aspect of the present invention, the damaged tissue accompanies acute kidney injury, chronic kidney disease, chronic renal failure, chronic glomerulonephritis, diabetic nephropathy, nephrosclerosis, rapidly progressive glomerulonephritis, polycystic kidney, tubulointerstitial nephritis, drug-induced nephritis, lupus nephritis, hydronephrosis, gouty kidney, cirrhosis, pulmonary fibrosis, a burn, interstitial pneumonia, drug-induced pneumonia, irradiation pneumonitis, chronic obstructive pulmonary disease, an acute respiratory distress syndrome, cartilage damage, a bone defect, spinal cord damage, periodontal disease, multiple sclerosis, rheumatoid arthritis, systemic lupus erythematosus, Crohn's disease, diabetes, arteriosclerosis, myocardial infarction, stroke, Alzheimer's disease, macular degeneration, viral hepatitis, alcoholic hepatitis, non-alcoholic steatohepatitis, jawbone reconstruction, cleft palate, a bone replacement material, a bone defect, a bone system disease, a dry eye, a corneal disorder, pharyngitis, arthritis, a cancer, cancer neighborhood fibrosis, or fibrosis.

### Advantageous Effects of Invention

According to an embodiment of the present invention, it is possible to provide a novel repair agent for damaged tissue that brings about a notably high effect of repairing damaged tissue, as compared with a repair agent for damaged tissue containing mesenchymal stem cells cultured with use of a serum-free medium at a normal oxygen concentration equivalent to the atmosphere, and to provide a method for producing such a repair agent.

### Brief Description of Drawings

Fig. 1 shows an image showing a result of detection of α-SMA by Western blotting in an Example of the present invention.
Fig. 2 shows an image showing a result of immunostaining carried out on α-SMA, collagen type I, and collagen type III in an Example of the present invention.
Fig. 3 shows an image showing a result of detection of α-SMA by Western blotting in an Example of the present invention.
Fig. 4 shows an image showing a result of detection of α-SMA by Western blotting in an Example of the present invention.
Fig. 5 shows graphs comparing expression amounts of surface antigens expressed on human bone marrow-derived mesenchymal stem cells which are used in a repair agent, in an Example of the present invention.
Fig. 6 shows graphs comparing VEGF expression amounts and comparing HGF expression amounts in human bone marrow-derived mesenchymal stem cells under conditions at various oxygen concentrations, in an Example of the present invention.
Fig. 7 shows a graph comparing VEGF expression amounts in human bone marrow-derived mesenchymal stem cells under conditions in various culture times, in an Example of the present invention.
Fig. 8 shows graphs comparing expression amounts of genes expressed in human bone marrow-derived mesenchymal stem cells, in an Example of the present invention.
Fig. 9 shows a graph comparing the numbers of human bone marrow-derived mesenchymal stem cells, in an Example of the present invention.
Fig. 10 shows graphs comparing VEGF expression amounts and comparing HGF expression amounts in human bone marrow-derived mesenchymal stem cells under conditions in various mediums and at various oxygen concentrations, in an Example of the present invention.

### Description of Embodiments

The following description will discuss an embodiment of the present invention. The present invention is not limited to arrangements described below, but may be altered in various ways by a skilled person within the scope of the claims. The present invention also encompasses, in its technical scope, any embodiment and any working example derived by appropriately combining technical means disclosed in differing embodiments or working examples. Moreover, all scientific literatures and patent literatures described in this specification are incorporated herein as reference literatures. Any numerical range expressed as "A to B" herein means "not less than A and not more than B" unless otherwise specified herein.

### [1. Method for producing repair agent]

A method of an embodiment of the present invention for producing a repair agent for damaged tissue (hereinafter also referred to as "a production method of an embodiment of the present invention") includes the step of culturing mesenchymal stem cells in a serum-free medium at an oxygen concentration of less than 5%.

The serum-free medium is not particularly limited in composition provided that the serum-free medium makes it possible to culture mesenchymal stem cells. The serum-free medium can be a publicly known serum-free medium as appropriate. Examples of the publicly known serum-free medium include STK1 (TWOCELLS COMPANY, LIMITED), STK2 (TWOCELLS COMPANY, LIMITED), a kit for a complete synthetic medium dedicated for human mesenchymal stem cells (MSCGM-CD BulletKit) (Lonza), Mesenchymal Stem Cell Growth Medium DXF (Ready-touse) (PromoCell GmbH.), Stem Pro MSC SFM Xeno free (Thermo Fisher Scientific Inc.), and MesenCult-ACF Medium Kit (STEMCELL Technologies Inc.).

Specific examples of the serum-free medium include (i) a serum-free medium containing an FGF, a PDGF, an EGF, at least one phospholipid, and at least one fatty acid, (ii) a serum-free medium containing an FGF, a PDGF, an EGF, a transforming growth factor-β (TGF-β), at least one phospholipid, and at least one fatty acid, (iii) a serum-free medium containing an FGF, a PDGF, an EGF, dexamethasone, insulin, serum albumin, at least one phospholipid, and at least one fatty acid, and (iv) a serum-free medium containing an FGF, a PDGF, an EGF, a TGF-β, dexamethasone, insulin, serum albumin, at least one phospholipid, and at least one fatty acid. The serum-free medium which contains at least any one of dexamethasone, insulin, and serum albumin brings about an effect of (i) extending a lifetime of mesenchymal stem cells and (ii) accelerating proliferation of mesenchymal stem cells.

Note that the serum-free medium (i) and the serum-free medium (iii) can each be a serum-free medium containing no TGF-β and/or no HGF. Note also that the serum-free medium (ii) and the serum-free medium (iv) can each be a serum-free medium containing no HGF.

The phospholipid is exemplified by, but not particularly limited to, phosphatidic acid, lysophosphatidic acid, phosphatidylinositol, phosphatidyl serine, phosphatidyl ethanolamine, phosphatidyl choline, and phosphatidyl glycerol. Those phospholipids can each be used alone, or two or more of those phospholipids can be used in combination (for example, phosphatidic acid and phosphatidyl choline can be used in combination). Those phospholipids can be derived from animals or plants.

The fatty acid is exemplified by, but not particularly limited to, linoleic acid, oleic acid, linolenic acid, arachidonic acid, myristic acid, palmitoyl acid, palmitic acid, and stearic acid. Those fatty acids can each be used alone, or two or more of those fatty acids can be used in combination.

A serum-free medium for use in an embodiment of the present invention optionally can contain component(s) different from the above-mentioned components, such as cholesterol and/or a hepatocyte growth factor (HGF). An HGF is contained at a final concentration of preferably 0.1 ng/ml to 50 ng/ml, and more preferably 5 ng/ml, relative to a basal medium (described later). It is a matter of course that such a component(s) is/are not essential to an aspect of the present invention.

The production method of an embodiment of the present invention is more specifically described below.

### (A. Pre-culture step)

In the production method of an embodiment of the present invention, mesenchymal stem cells can be cultured in a serum-free medium (e.g., a serum-free medium containing an FGF, a PDGF, a TGF-β, an EGF, at least one phospholipid, and at least one fatty acid, the serum-free medium more preferably further containing at least any one of dexamethasone, insulin, and serum albumin) (hereinafter referred to as "pre-culture step"). Note that the pre-culture step is not essential to the present invention. According to the arrangement, it is possible to efficiently obtain a large number of mesenchymal stem cells whose ability to repair tissue damage is improved. Note that the serum-free medium can be a serum-free medium containing no HGF or can be a basal medium such as a DMEM medium.

The oxygen concentration at which mesenchymal stem cells are cultured in the pre-culture step is not particularly limited. For example, the oxygen concentration can be 21% or less, or the oxygen concentration can be 5% or less. A lower limit of the oxygen concentration is not particularly limited, and can be, for example, 1%, 3%, 5%, 10%, or 20%.

A basal medium for constituting the serum-free medium for use in the pre-culture step is not limited to any particular basal medium provided that the basal medium is a medium for an animal cell which medium is well known in the present field. Preferable examples of the basal medium include a Ham's F12 medium, a DMEM medium, an RPMI-1640 medium, and an MCDB medium. Those basal media can each be used alone, or a mixture of two or more of those basal media can be used. In an embodiment, a basal medium for constituting the serum-free medium is preferably a medium in which MCDB and DMEM are mixed at a ratio of 1:1. In an embodiment, the serum-free medium which is obtained by adding an FGF, a PDGF, an EGF, at least one phospholipid, and at least one fatty acid to the basal medium can be used in the pre-culture step.

An FGF is contained at a final concentration of preferably 0.1 ng/ml to 100 ng/ml, and more preferably 3 ng/ml, relative to the basal medium. A PDGF is contained at a final concentration of preferably 0.5 ng/ml to 100 ng/ml, and more preferably 10 ng/ml, relative to the basal medium. An EGF is contained at a final concentration of preferably 0.5 ng/ml to 200 ng/ml, and more preferably 20 ng/ml, relative to the basal medium. A phospholipid(s) is/are contained in total at a final concentration of preferably 0.1 pg/ml to 30 pg/ml, and more preferably 10 pg/ml, relative to the basal medium. A total amount of a fatty acid(s) contained relative to the basal medium is preferably 1/1000 to 1/10, and more preferably 1/100 of a weight of the basal medium.

Dexamethasone is contained at a final concentration of preferably 10⁻¹⁰ M to 10⁻⁵ M, and more preferably 10⁻⁹ M to 10⁻⁶ M, relative to the basal medium. Insulin is contained at a final concentration of preferably 0.01 µg/ml to 500 pg/ml, and more preferably 0.1 pg/ml to 50 pg/ml, relative to the basal medium. Serum albumin is contained at a final concentration of preferably 0.01 mg/ml to 50 mg/ml, and more preferably 0.1 mg/ml to 5 mg/ml, relative to the basal medium.

By using such a serum-free medium, it is possible to obtain a proliferation promoting effect equal to or greater than that of a serum-containing medium while preventing a heteroprotein from being mixed into the serum-free medium. This allows mesenchymal stem cells to be proliferated as desired.

A phospholipid and a fatty acid each contained in the serum-free medium can be a specific phospholipid and a specific fatty acid, respectively, each of which has already been described.

As used herein, an FGF means a growth factor selected from a fibroblast growth factor (FGF) family, and is preferably an FGF-2 (bFGF). Alternatively, an FGF can be, for example, an FGF-1 selected from another FGF family. As used herein, a PDGF means a growth factor selected from a platelet derived growth factor (PDGF) family, and is preferably a PDGF-BB or a PDGF-AB.

An EGF means a growth factor selected from an epidermal growth factor (EGF) family.

In an embodiment, the serum-free medium can further contain at least two factors selected from the group consisting of a connective tissue growth factor (CTGF), a vascular endothelial growth factor (VEGF), and an ascorbic acid compound.

As used herein, an ascorbic acid compound means ascorbic acid (vitamin C), ascorbate diphosphate, or a compound similar to ascorbic acid or ascorbate diphosphate.

In an aspect, the serum-free medium preferably contains a lipid antioxidant. In an embodiment, the lipid antioxidant contained in the serum-free medium can be DL-α-tocopherol acetate (vitamin E). The serum-free medium can further contain a surfactant. In an embodiment, the surfactant contained in the serum-free medium can be Pluronic F-68 or Tween 80.

The serum-free medium can further contain insulin, transferrin, dexamethasone, serum albumin, and selenate. As used herein, insulin can be an insulin-like growth factor, can be derived from a natural cell, or can be produced by genetic modification.

In the pre-culture step, mesenchymal stem cells isolated from animal tissues such as human tissues by a conventionally publicly-known method are seeded into the serum-free medium (described earlier) and are cultured until the number of mesenchymal stem cells proliferated reaches the desired number of mesenchymal stem cells (it is a matter of course that in the pre-culture step, the mesenchymal stem cells can alternatively be merely maintained in the serum-free medium without being proliferated). As culture conditions, it is preferable that mesenchymal stem cells separated from 1 mg to 500 mg of a tissue piece(s) (containing mesenchymal stem cells) are seeded per 1 ml of a medium, a culture temperature is 37°C±1°C, and a carbon dioxide (CO₂) concentration in the environment for culturing mesenchymal stem cells is 10% or less.

In an embodiment, a TGF-β is contained at a final concentration of preferably 0.5 ng/ml to 100 ng/ml, and more preferably 10 ng/ml, relative to the basal medium.

In the pre-culture step, mesenchymal stem cells are seeded and cultured until the number of mesenchymal stem cells proliferated reaches the desired number of mesenchymal stem cells. As culture conditions, it is preferable that 1 to 2×10⁴ mesenchymal stem cells are seeded per 1 ml of a medium, a culture temperature is 37°C±1°C, and a carbon dioxide (CO₂) concentration in the environment for culturing mesenchymal stem cells is 5% or less.

A cell culture time is not particularly limited, provided that the cell culture time is long enough to obtain an intended number of cells. For example, the culture time can be 1 hour to 5 hours, 1 hour to 10 hours, 1 hour to 20 hours, 1 hour to 1 day, 1 hour to 10 days, 1 hour to 30 days, or 1 hour to 50 days.

In the pre-culture step, a culture vessel for use in culture is not limited to any particular culture vessel provided that the culture vessel allows the mesenchymal stem cells to be proliferated. Preferable examples of the culture vessel include a 75 cm² flask (manufactured by Falcon) and a 75 cm² flask (manufactured by SUMITOMO BAKELITE CO., LTD.). Note, however, that culture of some cells may be affected by a type of a culture vessel used. Thus, in order to more efficiently culture the mesenchymal stem cells, it is preferable to use a culture vessel suitable for culture to carry out the pre-culture step with respect to each type of mesenchymal stem cells to be cultured (hereinafter also referred to as "culture target cells").

The mesenchymal stem cells which are to be subjected to the pre-culture step are preferably exemplified by, but not particularly limited to, initial mesenchymal stem cells, i.e., cells which have never been subcultured since the cells were collected from animal tissues such as human tissues.

Moreover, in order to more efficiently proliferate the mesenchymal stem cells in the pre-culture step, it is preferable to use a culture vessel suitable for culture to carry out the pre-culture step with respect to each type of mesenchymal stem cells to be cultured in the pre-culture step (hereinafter also referred to as "pre-culture target cells"). Examples of a method for selecting a culture vessel suitable for culture of the pre-culture step target cells include a method in which an optimum culture vessel is selected by pre-culture step target cells. Specifically, a plurality of types of culture vessels are prepared, and pre-culture step target cells are proliferated under the same condition except that the culture vessels differ in type. After two weeks have elapsed from the start of culture, the numbers of cells are measured by a publicly-known method. Then, it can be determined that a culture vessel holding the most cells is the most suitable for culture of the pre-culture step target cells. Further, in a case where the pre-culture step target cells are proliferated at a high speed, it can be determined, even before two weeks have elapsed from the start of culture, that a culture vessel in which the number of pre-culture step target cells reaches 80% to 90% of the number of cells in a confluent state in the shortest period is the most suitable for culture of the pre-culture step target cells.

In the pre-culture step of the production method of an embodiment of the present invention, in a case where a culture vessel suitable for proliferation of the pre-culture step target cells has been made clear, it is possible to use that culture vessel. In contrast, in a case where a culture vessel suitable for culture of the pre-culture step target cells has not been made clear, the production method of an embodiment of the present invention can further include, before the pre-culture step, a "culture vessel selecting step" for selecting a culture vessel suitable for culture of the pre-culture step target cells.

Note that adhesion of the mesenchymal stem cells to a culture vessel is a requisite to proliferation of the mesenchymal stem cells. Thus, in a case where the mesenchymal stem cells less strongly adhere to the culture vessel, the serum-free medium is preferably caused, in the pre-culture step, to further contain cell adhesion molecules. Examples of the "cell adhesion molecules" include fibronectin, collagen, and gelatin. Those cell adhesion molecules can be used alone in one kind or can be used in combination of two or more kinds.

In the pre-culture step, the mesenchymal stem cells can be subcultured at least once. The mesenchymal stem cells are anchorage-dependently proliferated. Thus, for example, in a case where the mesenchymal stem cells are locally unevenly proliferated, by subculturing the mesenchymal stem cells in the process of carrying out the pre-culture step, it is possible to culture the mesenchymal stem cells under a better condition. Note that it is preferable that the pre-culture step is carried out during a period from primary culture (P0) to third passage culture (P3).

A method of subculturing the mesenchymal stem cells is not particularly limited, and the mesenchymal stem cells can be subcultured by a conventionally publicly-known subculturing method. In order that mesenchymal stem cells which have been subcultured will be in good condition, the mesenchymal stem cells which are being subcultured in the pre-culture step are preferably detached with use of a cell detaching agent containing neither a mammal-derived component nor a microorganism-derived component. Examples of the "cell detaching agent containing neither a mammal-derived component nor a microorganism-derived component" include ACCUTASE (Innovative Cell Technologies, Inc.).

The following description discusses an example of a subculture method which is carried out by using ACCUTASE as the "cell detaching agent containing neither a mammal-derived component nor a microorganism-derived component". The mesenchymal stem cells are detached and subcultured by a process including the steps (i) to (vi) below. Note that the following describes a subculture method which is carried out by using a T-25 flask (Falcon) as a culture vessel.

(i) A cell layer on the flask is washed with 5 mL of PBS (-).
(ii) To the cell layer, 2 mL of ACCUTASE is added.
(iii) The cell layer is caused to stand still at a room temperature for approximately two minutes, and after it is confirmed that cells have been detached from the flask, the PBS (-) which contains the cells thus detached is transferred to a centrifuging tube.
(iv) 7 mL of PBS (-) is added to the flask so that a bottom surface of the flask is rinsed with that PBS (-).
(v) A resultant solution obtained in the step (iv) is transferred to the centrifuging tube used in the step (iii), and the solution is centrifuged at 1500 rpm (200 ×g to 1000 ×g) for five minutes.
(vi) Supernatant is removed from the centrifuging tube, and cells are seeded into a serum-free medium in a new flask at a seeding density of 5,000 cells/cm².

### (B. Low-oxygen culture step)

In the production method of an embodiment of the present invention, after the pre-culture step, mesenchymal stem cells are cultured in a serum-free medium (e.g., a serum-free medium containing an FGF, a PDGF, an EGF, at least one phospholipid, and at least one fatty acid, the serum-free medium more preferably further containing at least any one of dexamethasone, insulin, and serum albumin) at an oxygen concentration of less than 5% (hereinafter referred to as "low-oxygen culture step"). Note that the serum-free medium can be a serum-free medium containing no TGF-β and/or no HGF or can be a serum-free medium containing TGF-β and/or HGF.

An oxygen concentration in a gas (hereinafter, referred to as "environment for culturing mesenchymal stem cells"), which can be in contact with mesenchymal stem cells being cultured (specifically, a medium for use in culture of mesenchymal stem cells), can be less than 5%, 4% or less, 3% or less, 2% or less, or 1% or less. The oxygen concentration in the gas is preferably 3% or less, more preferably 1% or less. A lower limit of the oxygen concentration in the gas is not particularly limited, and can be set to, for example, higher than 0%. The lower limit of the oxygen concentration in the gas only needs to be a concentration at which mesenchymal stem cells can be grown and do not die out.

In a case where the oxygen concentration in the environment for culturing mesenchymal stem cells is less than 5%, the effect by the cultured mesenchymal stem cells of repairing damaged tissue (e.g., anti-fibrosis effect, effect of suppressing infiltration of inflammatory cells, effect of controlling macrophage activity) is enhanced.

The environment for culturing mesenchymal stem cells can contain a gas component different from oxygen. The gas component is not particularly limited, provided that mesenchymal stem cells can be grown without dying out in the gas component. The gas component can be a gas component different from oxygen contained in the atmosphere (e.g., nitrogen, carbon dioxide, argon, or a mixture thereof).

In this specification, the symbol "%" indicating the concentration of the gas component such as oxygen more specifically means "% by volume".

In regard to the gas for use in culture of mesenchymal stem cells, it is possible that a device such as an incubator that is typically used for culture of mesenchymal stem cells is filled with the gas, it is possible that a hermetic container is provided inside a device such as an incubator and the container is filled with the gas, or it is possible that a hermetic container (e.g., a chamber) is provided to surround a device such as an incubator, and the hermetic container is filled with the gas. Note that the gas preferably contains approximately 5% of carbon dioxide (CO₂) in order to adjust pH of the medium.

Here, the "serum-free medium" in the low-oxygen culture step can contain TGF-β. The serum-free medium used in the pre-culture step and the serum-free medium used in the low-oxygen culture step may be herein referred to as a "serum-free medium A" and a "serum-free medium B", respectively. Since components except a TGF-β (e.g., an FGF, a PDGF, an EGF, dexamethasone, insulin, serum albumin, at least one phospholipid, and at least one fatty acid) and a basal medium are as described earlier in (A. Pre-culture step) in which the serum-free medium A is discussed, a description thereof is omitted here. Furthermore, respective amounts of the above components contained in the serum-free medium A used in the pre-culture step can be identical to the respective amounts (described earlier) of the above components contained in the serum-free medium B. Note that the serum-free medium B can be used as the serum-free medium A.

A culture time in the low-oxygen culture step is not limited to any particular culture time provided that the culture time allows the number of the mesenchymal stem cells to reach an intended number of cells. For example, the culture time can be 6 hours to 12 hours, 6 hours to 24 hours (1 day), 6 hours to 48 hours, 12 hours to 24 hours, 24 hours to 48 hours, and is preferably 12 hours or more, further preferably 24 hours.

Since a method for selecting a culture vessel suitable for proliferation of low-oxygen culture step target cells is as described earlier in (A. Pre-culture step), a description thereof is omitted here.

In a case where the mesenchymal stem cells less strongly adhere to the culture vessel, the serum-free medium B can further contain cell adhesion molecules. Since the cell adhesion molecules are as described earlier in (A. Pre-culture step), a description thereof is omitted here.

In the low-oxygen culture step, the mesenchymal stem cells can be subcultured at least once. By subculturing the mesenchymal stem cells in the process of carrying out the low-oxygen culture step, it is possible to culture the mesenchymal stem cells under a better condition.

Note that mesenchymal stem cells which have been subcultured at least once (P1) since the mesenchymal stem cells were collected from animal tissues such as human tissues are preferably subjected to the low-oxygen culture step.

Since (i) a method for subculturing the mesenchymal stem cells in the process of carrying out the low-oxygen culture step and (ii) a subculture method carried out in a case where cells which have been obtained through the pre-culture step are subjected to the low-oxygen culture step are as described earlier in (A. Pre-culture step), a description thereof is omitted here.

The production method of an embodiment of the present invention can further include, before the low-oxygen culture step (or before the pre-culture step), a culture vessel selecting step of selecting a culture vessel suitable for proliferation of mesenchymal stem cells. Since a method for selecting a culture vessel suitable for proliferation of mesenchymal stem cells is as described earlier in (A. Pre-culture step), a description thereof is omitted here.

### (C. Screening step)

The production method of an embodiment of the present invention can optionally further include, after the pre-culture step and/or the low-oxygen culture step, a screening step of selecting, from the mesenchymal stem cells by screening, mesenchymal stem cells which are non-tumorigenic.

By using, as a repair agent, the mesenchymal stem cells which have been selected in the screening step, it is possible to achieve a repair agent containing mesenchymal stem cells which are non-tumorigenic, and consequently to achieve a safer repair agent.

In the screening step, it can be investigated by a tumorigenicity test method with use of an in vivo sensitive immunodeficient mouse (NOG mouse) whether the mesenchymal stem cells are tumorigenic.

Specifically, mesenchymal stem cells (e.g., 1,000,000 cells) cultured in the serum-free medium at the oxygen concentration of less than 5% (described earlier) and Hela cells (e.g., 1,000 cells) are subcutaneously-implanted in 10 places in an NOG mouse. In a case where the tumorigenicity of the mesenchymal stem cells cultured in the serum-free medium is not observed under a condition (e.g., a specific mouse breeding time) in which the tumorigenicity of the Hela cells (specifically, development of a tumor at a place at which the Hela cells have been implanted) can be observed, it is possible to determine that those mesenchymal stem cells are non-tumorigenic.

### [2. Repair agent for damaged tissue]

A repair agent for damaged tissue in accordance with an embodiment of the present invention (hereinafter also referred to as "repair agent of an embodiment of the present invention") contains mesenchymal stem cells in which a gene that encodes angiopoietin-like 4 (or a homologue thereof), a gene that encodes fatty acid binding protein 3 (or a homologue thereof), a gene that encodes delta-like 2 homolog (Drosophila), a gene that encodes fructose-bisphosphate aldolase C (or a homologue thereof), a gene that encodes TRPM8 channel-associated factor 2 (or a homologue thereof), and a gene that encodes REST corepressor 2 (or a homologue thereof) are respectively expressed in amounts of at least 3 or more times (preferably, independently for each gene, 3 or more times, 4 or more times, 5 or more times, 10 or more times, 20 or more times, 40 or more times, or 50 or more times) as compared with amounts in mesenchymal stem cells which have been cultured in a serum-containing medium at an oxygen concentration of 5% or more.

The repair agent of an embodiment of the present invention contains mesenchymal stem cells in which a gene that encodes angiopoietin-like 4 (or a homologue thereof), a gene that encodes fatty acid binding protein 3 (or a homologue thereof), a gene that encodes delta-like 2 homolog (Drosophila), a gene that encodes fructose-bisphosphate aldolase C (or a homologue thereof), a gene that encodes TRPM8 channel-associated factor 2 (or a homologue thereof), and a gene that encodes REST corepressor 2 (or a homologue thereof) are respectively expressed in amounts of at least twice or more (preferably, independently for each gene, 3 or more times, 4 or more times, 5 or more times, 10 or more times, or 15 or more times) as compared with amounts in mesenchymal stem cells which have been cultured in a serum-containing medium at an oxygen concentration of 1% or less.

The repair agent of an embodiment of the present invention contains mesenchymal stem cells in which a gene that encodes angiopoietin-like 4 (or a homologue thereof), a gene that encodes fatty acid binding protein 3 (or a homologue thereof), a gene that encodes delta-like 2 homolog (Drosophila), a gene that encodes fructose-bisphosphate aldolase C (or a homologue thereof), a gene that encodes TRPM8 channel-associated factor 2 (or a homologue thereof), and a gene that encodes REST corepressor 2 (or a homologue thereof) are respectively expressed in amounts of twice or more (preferably, independently for each gene, 3 or more times, 4 or more times, 5 or more times, 7 or more times, or 10 or more times) as compared with amounts in mesenchymal stem cells which have been cultured in a serum-free medium at an oxygen concentration of 5% or more. In other words, the repair agent for damaged tissue in accordance with an embodiment of the present invention can contain mesenchymal stem cells cultured in a serum-free medium at an oxygen concentration of less than 5%.

The angiopoietin-like 4 is a protein involved in carbohydrate metabolism and lipid metabolism, and is encoded by an ANGPTL4 gene in a human.

The fatty acid binding protein 3 is a fatty acid binding protein, and is encoded by an FABP3 gene in a human.

The delta-like 2 homolog (Drosophila) is an epidermal growth factor-like protein, and is encoded by a DLK2 gene.

The Fructose-Bisphate Aldolase C is aldolase C, and is encoded by an ALDOC gene in a human.

The TRPM8 channel-associated factor 2 is a protein that adjusts transient receptor potential melastatin member 8 (TRPM8), and is encoded by a TCAF2 gene in a human.

The REST corepressor 2 is a protein that adjusts gene expression in a neuron, and is encode by an RCOR2 gene in a human.

Note here that the repair agent of an embodiment of the present invention means a pharmaceutical agent which has an effect of promoting repair of tissue damage. The repair agent of an embodiment of the present invention can also be referred to as (i) a pharmaceutical agent for suppressing fibrosis of a living tissue (i.e., a fibrosis suppressing agent), (ii) a pharmaceutical agent for suppressing infiltration of an inflammatory cell (i.e., an inflammatory cell infiltration suppressing agent), or (iii) a pharmaceutical agent for controlling activity of a macrophage (i.e., a macrophage activity controlling agent).

The expression "suppressing fibrosis of a living tissue" herein means suppressing tissue hardening caused by (i) abnormal proliferation of a connective tissue or (ii) abnormal accumulation of collagen in a connective tissue. For example, in a case where a living tissue suffers damage, a connective tissue constituting the living tissue may be abnormally proliferated in the process of recovery from the damage. The expression "suppressing fibrosis of a living tissue" means, for example, suppressing, after a living tissue has suffered damage, tissue hardening caused by (i) abnormal proliferation of a connective tissue constituting the living tissue or (ii) abnormal accumulation of collagen in a connective tissue.

The expression "suppressing infiltration of an inflammatory cell" herein means suppressing (i) destruction of a tissue by inflammatory cells (e.g., a macrophage, a lymphocyte, and a neutrophil) or (ii) proliferation of inflammatory cells (e.g., a macrophage, a lymphocyte, and a neutrophil) which enter a tissue.

The expression "controlling macrophage activity" herein means changing a phenotype of a macrophage. The expression "controlling macrophage activity" means, for example, changing a macrophage from a Pro-inflammatory phenotype (M1-type) macrophage which accelerates inflammation of a tissue into an Immune-regulatory phenotype (M2-type) macrophage. By changing a macrophage from an M1-type macrophage into an M2-type macrophage, it is possible to, for example, relieve inflammation of a tissue.

Mesenchymal stem cells can be cultured by (i) culturing mesenchymal stem cells only in a single serum-free medium or (ii) culturing mesenchymal stem cells in a plurality of serum-free media. Mesenchymal stem cells can be cultured in a plurality of serum-free media by, for example, culturing mesenchymal stem cells in a desired serum-free medium, then replacing the desired serum-free medium with another serum-free medium, and further culturing mesenchymal stem cells in the another serum-free medium.

Since the composition of the serum-free medium is as described earlier in [1. Method for producing repair agent], a description thereof is omitted here.

Mesenchymal stem cells for use in the repair agent of an embodiment of the present invention include not only mesenchymal stem cells isolated from tissues such as bone marrow, adipose tissue, a synovial membrane, a dental pulp, and a periodontal membrane, but also mesenchymal stem cells isolated from tissues such as a placenta, cord blood, and an umbilical cord. In view of providing a repair agent having higher repairing effect, it is preferable to employ mesenchymal stem cells isolated from adipose tissue. The mesenchymal stem cells for use in the repair agent of an embodiment of the present invention can be human mesenchymal stem cells (e.g., collected human mesenchymal stem cells) or a rat. A living tissue to be treated with the repair agent is exemplified by, but not particularly limited to, a kidney, a liver, a lung, a skin, a cartilage, a bone, the spinal cord, a tooth, a joint, blood vessels (an artery and a vein), a heart, a brain, a jaw, a mouth, an eye, a cornea, and a pharynx. That is, the repair agent can be intended to repair damage to such a living tissue.

Mesenchymal stem cells for use in an aspect of the present invention have a tissue fibrosis suppressing effect, an inflammatory cell infiltration suppressing effect, and an effect of changing an inflammation-accelerating macrophage (M1) into an inflammation-suppressing macrophage (M2), which are much more powerful than those of mesenchymal stem cells cultured in a serum-free medium at a normal oxygen concentration equivalent to the atmosphere.

A condition on which the repair agent works and which is accompanied by tissue damage is not limited to any particular condition. Examples of the condition include acute kidney injury, chronic kidney disease, chronic renal failure, chronic glomerulonephritis (such as IgA nephropathy, membranous nephropathy, membranoproliferative glomerulonephritis, focal glomerular sclerosis), other kidney diseases (e.g., diabetic nephropathy, nephrosclerosis, rapidly progressive glomerulonephritis, polycystic kidney, tubulointerstitial nephritis, drug-induced nephritis, lupus nephritis, hydronephrosis, gouty kidney), cirrhosis, pulmonary fibrosis, a burn, interstitial pneumonia, drug-induced pneumonia, irradiation pneumonitis, chronic obstructive pulmonary disease, an acute respiratory distress syndrome, cartilage damage, a bone defect, spinal cord damage, periodontal disease, multiple sclerosis, rheumatoid arthritis, systemic lupus erythematosus, Crohn's syndrome, diabetes, arteriosclerosis, myocardial infarction, stroke, Alzheimer's disease, macular degeneration, viral hepatitis, alcoholic hepatitis, non-alcoholic steatohepatitis, jawbone reconstruction, cleft palate, a bone replacement material, a bone defect, a bone system disease, a dry eye, a corneal disorder, pharyngitis, arthritis, a cancer, cancer neighborhood fibrosis, and fibrosis.

The repair agent of an embodiment of the present invention can contain a pharmaceutically acceptable additive(s) (e.g., a buffer, an antioxidant, a thickener, and/or an excipient), in addition to mesenchymal stem cells in which a gene that encodes angiopoietin-like 4, a gene that encodes fatty acid binding protein 3, a gene that encodes delta-like 2 homolog (Drosophila), a gene that encodes fructose-bisphosphate aldolase C, a gene that encodes TRPM8 channel-associated factor 2, and a gene that encodes REST corepressor 2 are respectively expressed in amounts of at least 3 or more times, as compared with respective expression amounts thereof in mesenchymal stem cells which have been cultured in a serum-containing medium at an oxygen concentration of 5% or more.

An amount of the additive(s) contained is not limited to any particular amount, but can be, for example, 0.01% by weight to 50% by weight, 0.01% by weight to 40% by weight, 0.01% by weight to 30% by weight, 0.01% by weight to 20% by weight, 0.01% by weight to 10% by weight, or 0.01% by weight to 1% by weight, of the repair agent of an embodiment of the present invention.

The number of mesenchymal stem cells contained in the repair agent of an embodiment of the present invention is not limited to any particular number and can be set as appropriate in accordance with a body weight of a subject to which the repair agent is to be administered. For example, 1×10² mesenchymal stem cells to 1×10¹⁰ mesenchymal stem cells, 1×10³ mesenchymal stem cells to 1×10¹⁰ mesenchymal stem cells, 1×10⁴ mesenchymal stem cells to 1×10¹⁰ mesenchymal stem cells, 1×10⁵ mesenchymal stem cells to 1×10¹⁰ mesenchymal stem cells, or 1×10⁶ mesenchymal stem cells to 1×10¹⁰ mesenchymal stem cells can be contained per dose (per administration). It is a matter of course that 10¹⁰ or more mesenchymal stem cells can be contained per dose (administration).

Examples of a method of administration of the repair agent include an intravascular injection, and local injections (e.g., an injection into the spinal cord (a spinally intracavitary injection), an intramuscular injection, a joint injection, and an injection into the brain).

A subject to which the repair agent for damaged tissue in accordance with an embodiment of the present invention is to be administered is not limited to any particular target. Examples of the subject to which the repair agent is to be administered include a human and mammals different from the human (e.g., cattle, a pig, a sheep, a goat, a horse, a dog, a cat, a rabbit, a mouse, and a rat).

An amount of administration of the repair agent of an embodiment of the present invention (for one administration) is not limited to any particular amount, but can be an amount that causes 1×10⁶ mesenchymal stem cells to 1×10⁷ mesenchymal stem cells, 1×10⁵ mesenchymal stem cells to 1×10⁸ mesenchymal stem cells, 1×10⁴ mesenchymal stem cells to 1×10⁹ mesenchymal stem cells to be administered per kilogram of body weight of the subject to which the repair agent is to be administered. The number of mesenchymal stem cells administered per human can be 1×10⁴ cells to 1×10⁹ cells. While taking into consideration the difference in area of body surface between a subject to be administered and a human, the number of mesenchymal stem cells per human can be 2×10³ cells to 2×10⁸ cells.

### [3. Other]

An aspect of the present invention can also be arranged as below. Note that a "method for repairing damaged tissue" described below can be a "method for preventing damaged tissue" or a "method for treating damaged tissue".

<1> A method for repairing damaged tissue, the method including the step of: administering, to a subject organism (e.g., a human or a mammal different from the human (such as cattle, a pig, a sheep, a goat, a horse, a dog, a cat, a rabbit, a mouse, or a rat)), a repair agent for damaged tissue, the repair agent containing mesenchymal stem cells in which a gene that encodes angiopoietin-like 4, a gene that encodes fatty acid binding protein 3, a gene that encodes delta-like 2 homolog (Drosophila), a gene that encodes fructose-bisphosphate aldolase C, a gene that encodes TRPM8 channel-associated factor 2, and a gene that encodes REST corepressor 2 are respectively expressed in amounts of at least 3 or more times, as compared with respective expression amounts thereof in mesenchymal stem cells which have been cultured in a serum-containing medium at an oxygen concentration of 5% or more.
<2> The method described in <1>, in which the repair agent is intended to (i) suppress fibrosis of a living tissue, (ii) suppress infiltration of an inflammatory cell, or (iii) control activity of a macrophage.
<3> The method described in <1> or <2>, in which: the damaged tissue accompanies acute kidney injury, chronic kidney disease, chronic renal failure, chronic glomerulonephritis, diabetic nephropathy, nephrosclerosis, rapidly progressive glomerulonephritis, polycystic kidney, tubulointerstitial nephritis, drug-induced nephritis, lupus nephritis, hydronephrosis, gouty kidney, cirrhosis, pulmonary fibrosis, a burn, interstitial pneumonia, drug-induced pneumonia, irradiation pneumonitis, chronic obstructive pulmonary disease, an acute respiratory distress syndrome, cartilage damage, a bone defect, spinal cord damage, periodontal disease, multiple sclerosis, rheumatoid arthritis, systemic lupus erythematosus, Crohn's disease, diabetes, arteriosclerosis, myocardial infarction, stroke, Alzheimer's disease, macular degeneration, viral hepatitis, alcoholic hepatitis, non-alcoholic steatohepatitis, jawbone reconstruction, cleft palate, a bone replacement material, a bone defect, a bone system disease, a dry eye, a corneal disorder, pharyngitis, arthritis, a cancer, cancer neighborhood fibrosis, or fibrosis.

### Examples

### [Example 1]

A kidney fibrosis suppressing effect brought about by implantation of mesenchymal stem cells cultured in a serum-free medium was studied.

### <Method: preparation of MSC>

In this Example 1, as MSC, human mesenchymal stem cells cultured with use of a serum-free medium (STK2 medium, TWOCELLS COMPANY, LIMITED: corresponding to the "serum-free medium containing an FGF, a PDGF, an EGF, at least one phospholipid, and at least one fatty acid") were used.

In Example, human bone marrow-derived mesenchymal stem cells (1%O₂hMSC) which had been cultured for 24 hours under a 1% oxygen environment were used. Meanwhile, in Comparative Example, human bone marrow-derived mesenchymal stem cells (21%O₂hMSC) which had been cultured for 24 hours under a 21% oxygen environment were used. Note that the culture of those human bone marrow-derived mesenchymal stem cells was carried out under the atmospheric pressure.

A gas, which had been prepared to contain oxygen (1% or 21%), carbon dioxide (5%), and nitrogen (94% or 74%) and accommodated in a gas bottle, was supplied from the gas bottle to the environment in which the human bone marrow-derived mesenchymal stem cells were cultured.

### <Method: preparation of model of disease>

A kidney fibrosis model was prepared by blocking the left renal artery of a Sprague Dawley rat (SD rat) (8-week old, male) for 60 minutes, and then releasing the blockage (renal ischemia-reperfusion injury (IRI)). The IRI was caused according to a known method.

### <Method: administration of test substance/placebo

The prepared 500,000 cells of MSC (21%O₂hMSC or 1%O₂hMSC) were suspended in 200 µL of PBS. The suspended MSC was administered into the blood vessel of the SD rat which had been subjected to the IRI (a group to which 21%O₂hMSC was administered may be referred to as "21%O₂hMSC group", and a group to which 1%O₂hMSC was administered may be referred to as "1%O₂hMSC group"). As a control, 200 µL of only PBS was administered into the blood vessel of the SD rat which had been subjected to the IRI (this group may be referred to as "PBS group").

For the "21%O₂hMSC group", the influence of the test substance was evaluated with use of n=2 (left kidneys taken from two SD rats subjected to the IRI). For the "PBS group" and the "1%O₂hMSC group", the influence of the test substance was evaluated with use of n=3 (left kidneys taken from three SD rats subjected to the IRI).

### <Method: sacrifice and material collection>

After 21 days had elapsed from the administration of the test substance (1%O₂hMSC, 21%O₂hMSC, or PBS), the left kidneys of the SD rats were collected. For the collected kidneys, kidney fibrosis and increase/decrease of extracellular matrix accompanying kidney fibrosis were evaluated by Western blotting with use of an anti-α-SMA antibody and immunostaining with use of an anti-α-SMA antibody, an anti-collagen type I antibody, and an anti-collagen type III antibody. Note that confirmation in the Western blotting was carried out in accordance with a well-known protocol of Western blotting. The immunostaining was carried out with use of commercially-available antibodies in accordance with a well-known protocol.

### <Results>

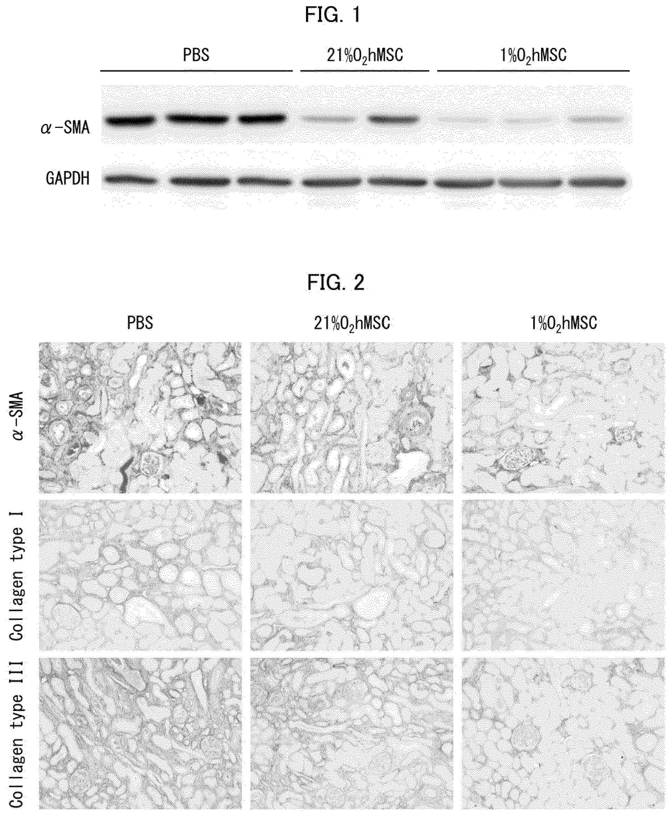

Fig. 1 shows the result of Western blotting using the anti-α-SMA antibody. Fig. 2 shows the result of immunostaining using the anti-α-SMA antibody, the anti-collagen type I antibody, and the anti-collagen type III antibody.

As shown in Fig. 1, for the "21%O₂hMSC group", expression of α-SMA, which is a molecular marker for kidney fibrosis, was suppressed as compared with the "PBS group". Furthermore, for the "1%O₂hMSC group", expression of α-SMA was further suppressed as compared with the "21%O₂hMSC group".

As shown in Fig. 2, the expression of α-SMA, which is a molecular marker for kidney fibrosis and was induced by subjecting the SD rat to the IRI, and the expression of the collagen type I and the collagen type III, which are the extracellular matrices whose expression is enhanced due to kidney fibrosis, were suppressed for the "21%O₂hMSC group" as compared with the "PBS group". Furthermore, for the "1%O₂hMSC group", the expressions of α-SMA, collagen type I, and collagen type III were further suppressed as compared with the "21%O₂hMSC group".

### <Conclusion of Example 1>

It was confirmed that the human bone marrow-derived mesenchymal stem cells (1%O₂hMSC group) cultured for 24 hours under the environment of low oxygen concentration had the enhanced effect of suppressing the progress of kidney fibrosis, as compared with the human bone marrow-derived mesenchymal stem cells (21%O₂hMSC group) cultured for 24 hours under the environment of normal oxygen concentration.

### [Example 2-1]

A fibrosis suppressing effect brought about by culture supernatant of human bone marrow-derived mesenchymal stem cells cultured in a serum-free medium was studied.

Fibrosis induction caused by TGF-β1 stimulation was carried out on human renal proximal tubular epithelial cells (HK2), and culture supernatant of the human bone marrow-derived mesenchymal stem cells (hereinafter, also referred to as "MSC-CM") was added to the human proximal tubular epithelial cells (HK2), and an expression level of α-SMA was evaluated.

### <Preparation of culture supernatant>

With use of an STK2 medium, human bone marrow-derived mesenchymal stem cells were cultured for 24 hours under a 21% oxygen environment. The STK2 medium was removed from the cultured matter to obtain human bone marrow-derived mesenchymal stem cells, and then the human mesenchymal stem cells were cultured for 24 hours under a 21% oxygen environment with use of a serum-free DMEM. After 24-hour culture, culture supernatant of the human bone marrow-derived mesenchymal stem cells was obtained as "21%O₂MSC-CM".

With use of an STK2 medium, human bone marrow-derived mesenchymal stem cells were cultured for 24 hours under a 1% oxygen environment. The STK2 medium was removed from the cultured matter to obtain human bone marrow-derived mesenchymal stem cells, and then the human bone marrow-derived mesenchymal stem cells were cultured for 24 hours under a 1% oxygen environment with use of a serum-free DMEM. After 24-hour culture, culture supernatant of the human bone marrow-derived mesenchymal stem cells was obtained as "1%O₂MSC-CM". Note that, for obtaining both "21%O₂MSC-CM" and "1%O₂MSC-CM", the human bone marrow-derived mesenchymal stem cells were cultured under the atmospheric pressure.

A gas, which had been prepared to contain oxygen (1% or 21%), carbon dioxide (5%), and nitrogen (94% or 74%) and accommodated in a gas bottle, was supplied from the gas bottle to the environment in which the human bone marrow-derived mesenchymal stem cells were cultured.

### <Evaluation Methods>

The human proximal tubular epithelial cells (HK2) were seeded to a 6-well plate at 1×10⁵ cells/well, cultured in a 10% bovine serum-containing DMEM medium (Sigma-Aldrich) for 18 hours. It was confirmed that the number of cells after the culture was sub-confluent (i.e., 80% to 90% of the number of cells in a confluent state). Then, the 10% bovine serum-containing DMEM medium was removed by sucking, and the HK2 was washed with PBS. The HK2 was seeded to a serum-free DMEM, and then the 21%O₂MSC-CM or the 1%O₂MSC-CM was added thereto. A resultant mixture was cultured for 24 hours under the atmosphere. After the 24-hour culture, TGF-β1 was added to the cultured matter. After the TGF-β1 was added to the cultured matter, a resultant mixture was cultured for another 12 hours. Then, expression of α-SMA in the HK2 was evaluated by Western blotting.

### <Results>

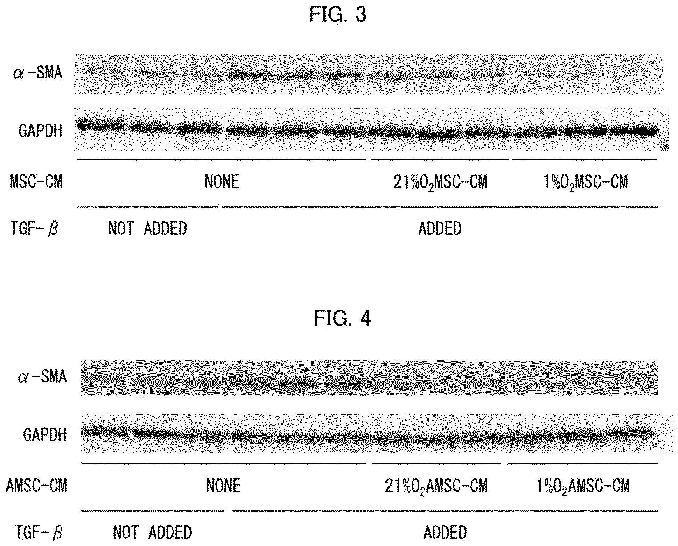

Fig. 3 shows the result of Western blotting using the anti-α-SMA antibody in the HK2. Fig. 3 shows: (i) an expression level of α-SMA in the case where culture supernatant and TGF-β1 were not added (serum-free DMEM was added and TGF-β1 was not added) (1st to 3rd lanes from the left in Fig. 3); (ii) an expression level of α-SMA in the case where culture supernatant was not added and TGF-β1 was added (serum-free DMEM and TGF-β1 were added) (4th to 6th lanes from the left in Fig. 3); (iii) an expression level of α-SMA in the case where 21%O₂MSC-CM was added as culture supernatant and TGF-β1 was further added (7th to 9th lanes from the left in Fig. 3); and (iv) an expression level of α-SMA in the case where 1%O₂MSC-CM was added as culture supernatant and TGF-β1 was further added (10th to 12th lanes from the left in Fig. 3).

As shown in Fig. 3, the expression level of α-SMA in the case where the serum-free DMEM and TGF-β1 were added was raised higher, as compared with the expression level of α-SMA in the case where the serum-free DMEM was added and TGF-β1 was not added. This shows that TGF-β can induce the human proximal tubular epithelial cells to be in the fibrosis state.

The expression level of α-SMA in the case where 21%O₂MSC-CM was added as culture supernatant and TGF-β1 was further added was found to be suppressed, as compared with the expression level of α-SMA in the case where the serum-free DMEM and TGF-β1 were added.

The expression level of α-SMA in the case where 1%O₂MSC-CM was added as culture supernatant and TGF-β1 was further added was found to be suppressed, as compared with the expression level of α-SMA in the case where 21%O₂MSC-CM was added as culture supernatant and TGF-β1 was further added.

### <Conclusion of Example 2-1>

The human bone marrow-derived mesenchymal stem cells (1%O₂MSC-CM) cultured for 24 hours or more in the low oxygen concentration (i.e., oxygen concentration of 1%) environment more strongly suppressed the fibrosis induction caused by TGF-β1 stimulation, as compared with the human bone marrow-derived mesenchymal stem cells (21%O₂MSC-CM) cultured under the environment at the oxygen concentration of 21%.

### [Example 2-2]

With use of human adipose-derived mesenchymal stem cells as MSC, a fibrosis suppressing effect brought about by culture supernatant of human adipose-derived mesenchymal stem cells cultured in a serum-free medium was studied, in a manner similar to that of [Example 2-1].

### <Preparation of culture supernatant>

With use of an STK2 medium, human adipose-derived mesenchymal stem cells were cultured for 24 hours under a 21% oxygen environment. The STK2 medium was removed from the cultured matter to obtain human adipose-derived mesenchymal stem cells, and then the human adipose-derived mesenchymal stem cells were cultured for 24 hours under a 21% oxygen environment with use of a serum-free DMEM. After 24-hour culture, culture supernatant of the human adipose-derived mesenchymal stem cells was obtained as "21%O₂AMSC-CM".

With use of an STK2 medium, human adipose-derived mesenchymal stem cells were cultured for 24 hours under a 1% oxygen environment. The STK2 medium was removed from the cultured matter to obtain human adipose-derived mesenchymal stem cells, and then the human adipose-derived mesenchymal stem cells were cultured for 24 hours under a 1% oxygen environment with use of a serum-free DMEM. After 24-hour culture, culture supernatant of the human adipose-derived mesenchymal stem cells was obtained as "1%O₂AMSC-CM". Note that, for obtaining both "21%O₂AMSC-CM" and "1%O₂AMSC-CM", the human adipose-derived mesenchymal stem cells were cultured under the atmospheric pressure.

A gas, which had been prepared to contain oxygen (1% or 21%), carbon dioxide (5%), and nitrogen (94% or 74%) and accommodated in a gas bottle, was supplied from the gas bottle to the environment in which the human adipose-derived mesenchymal stem cells were cultured.

### <Evaluation Methods>

The human proximal tubular epithelial cells (HK2) were seeded to a 6-well plate at 1×10⁵ cells/well, cultured in a 10% bovine serum-containing DMEM medium (Sigma-Aldrich) for 18 hours. It was confirmed that the number of cells after the culture was sub-confluent (i.e., 80% to 90% of the number of cells in a confluent state). Then, the 10% bovine serum-containing DMEM medium was removed by sucking, and the HK2 was washed with PBS. The HK2 was seeded to a serum-free DMEM, and then the 21%O₂AMSC-CM or the 1%O₂AMSC-CM was added thereto. A resultant mixture was cultured for 24 hours under the atmosphere. After the 24-hour culture, TGF-β1 was added to the cultured matter. After the TGF-β1 was added to the cultured matter, a resultant mixture was cultured for another 12 hours. Then, expression of α-SMA in the HK2 was evaluated by Western blotting.

### <Results>

Fig. 4 shows the result of Western blotting using the anti-α-SMA antibody in the HK2. Fig. 4 shows: (i) an expression level of α-SMA in the case where culture supernatant and TGF-β1 were not added (serum-free DMEM was added and TGF-β1 was not added) (1st to 3rd lanes from the left in Fig. 4); (ii) an expression level of α-SMA in the case where culture supernatant was not added and TGF-β1 was added (serum-free DMEM and TGF-β1 were added) (4th to 6th lanes from the left in Fig. 4); (iii) an expression level of α-SMA in the case where 21%O₂AMSC-CM was added as culture supernatant and TGF-β1 was further added (7th to 9th lanes from the left in Fig. 4); and (iv) an expression level of α-SMA in the case where 1%O₂AMSC-CM was added as culture supernatant and TGF-β1 was further added (10th to 12th lanes from the left in Fig. 4).

As shown in Fig. 4, the expression level of α-SMA in the case where the serum-free DMEM and TGF-β1 were added was raised higher, as compared with the expression level of α-SMA in the case where the serum-free DMEM was added and TGF-β1 was not added. This shows that TGF-β can induce the human proximal tubular epithelial cells to be in the fibrosis state.

The expression level of α-SMA in the case where 21%O₂AMSC-CM was added as culture supernatant and TGF-β1 was further added was found to be suppressed, as compared with the expression level of α-SMA in the case where the serum-free DMEM and TGF-β1 were added.

The expression level of α-SMA in the case where 1%O₂AMSC-CM was added as culture supernatant and TGF-β1 was further added was found to be suppressed to a degree equivalent to the expression level of α-SMA in the case where 21%O₂AMSC-CM was added as culture supernatant and TGF-β1 was further added.

### <Conclusion of Example 2-2>

The human adipose-derived mesenchymal stem cells (21%O₂AMSC-CM) cultured in the environment at the oxygen concentration of 21% and the human adipose-derived mesenchymal stem cells (1%O₂AMSC-CM) cultured for 24 hours or more in the low oxygen concentration (i.e., oxygen concentration of 1%) environment both strongly suppressed the fibrosis induction caused by TGF-β1 stimulation. That is, the human adipose-derived mesenchymal stem cells (21%O₂AMSC-CM) cultured in the environment at the oxygen concentration of 21% and the human adipose-derived mesenchymal stem cells (1%O₂AMSC-CM) cultured for 24 hours or more in the low oxygen concentration (i.e., oxygen concentration of 1%) environment both returned the expression level of α-SMA, which had been increased by the TGF-β1 stimulation, to the normal expression level of α-SMA.

As already shown in [Example 2-1], the mesenchymal stem cells cultured in the environment at the oxygen concentration of 21% have a weaker effect of suppressing fibrosis induction caused by TGF-β1 stimulation, as compared with the mesenchymal stem cells cultured in the low oxygen concentration (i.e., oxygen concentration of 1%) environment. In this [Example 2-2], even the human adipose-derived mesenchymal stem cells (21%O₂AMSC-CM), which had been cultured in the environment at the oxygen concentration of 21% and had a weaker effect of suppressing fibrosis induction caused by TGF-β1 stimulation, returned the expression level of α-SMA, which had been increased by the TGF-β1 stimulation, to the normal expression level of α-SMA. This shows that the human adipose-derived mesenchymal stem cells have the stronger effect of suppressing fibrosis induction caused by TGF-β1 stimulation, as compared with the human bone marrow-derived mesenchymal stem cells.

Since the 1%O₂AMSC-CM and 21%O₂AMSC-CM strongly suppressed fibrosis induction caused by TGF-β1 stimulation, it has been shown that the strong fibrosis suppressing effect by the human adipose-derived mesenchymal stem cells is maintained at high level even when the cells were cultured in the low oxygen environment.

### [Example 3]

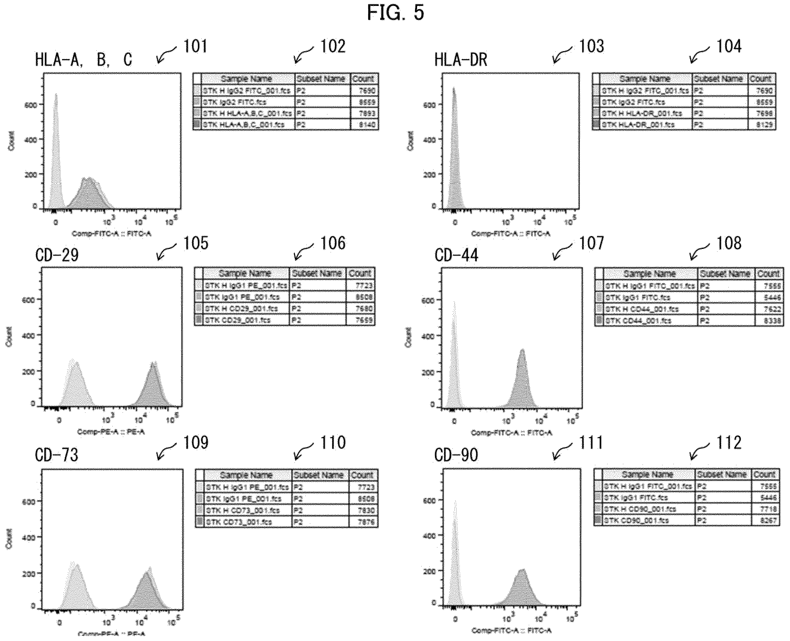

Fig. 5 shows the result of comparing expression amounts of surface antigens of human bone marrow-derived mesenchymal stem cells, which were expressed in (i) human bone marrow-derived mesenchymal stem cells (hereinafter, also referred to as "STK") cultured for 24 hours in an oxygen environment equivalent to the atmosphere (in other words, an environment in which a gas component was not prepared) with use of an STK2 medium and in (ii) human bone marrow-derived mesenchymal stem cells (hereinafter, also referred to as "STK H") cultured at an oxygen concentration equivalent to the atmosphere (in other words, an environment in which a gas component was not prepared) with use of an STK2 medium and then cultured for 24 hours in a 1% oxygen environment.

A gas, which had been prepared to contain oxygen (1%), carbon dioxide (5%), and nitrogen (94%) and accommodated in a gas bottle, was supplied from the gas bottle to the environment in which the human mesenchymal stem cells were cultured.

### <Comparison method>

With use of an anti-HLA-A,B,C antibody (BioLegend, San Diego, CA) which is of the human leukocyte antigen (HLA) class I, an anti-HLA-DR antibody (BioLegend) of HLA class II, and an anti-CD-29 antibody (BioLegend), an anti-CD-44 antibody (BioLegend), an anti-CD-73 antibody (BioLegend), an anti-CD-90 antibody (BioLegend), and an anti-CD-44 antibody (BioLegend) which are mesenchymal stem cell markers, expression amounts of surface antigens were analyzed by flow cytometry with use of BD FACSVerse (Becton, Dickinson and Company, Franklin Lakes, NJ).

### <Results>

In Fig. 5, expression amounts of the HLA-A,B,C antibody are shown in the graph 101, and the expression amounts of the HLA-A,B,C antibody are also shown in the counted values 102. Expression amounts of the HLA-DR antibody are shown in the graph 103, and the expression amounts of the HLA-DR antibody are also shown in the counted values 104. Expression amounts of the CD-29 antibody are shown in the graph 105, and the expression amounts of the CD-29 antibody are also shown in the counted values 106. Expression amounts of the CD-44 antibody are shown in the graph 107, and the expression amounts of the CD-44 antibody are also shown in the counted values 108. Expression amounts of the CD-73 antibody are shown in the graph 109, and the expression amounts of the CD-73 antibody are also shown in the counted values 110. Expression amounts of the CD-90 antibody are shown in the graph 111, and the expression amounts of the CD-90 antibody are also shown in the counted values 112.

As shown in the graph 101 indicating the expression amounts of the HLA-A,B,C antibody and the counted values 102 indicating the expression amounts of the HLA-A,B,C antibody, the expression amount of the anti-HLA-A,B,C antibody expressed in the STK was equivalent to that of the HLA-A,B,C antibody expressed in the STK H. As shown in the graph 103 indicating the expression amounts of the HLA-DR antibody and the counted values 104 indicating the expression amounts of the HLA-DR antibody, the HLA-DR antibody was not expressed in STK and STK H. Furthermore, expressions of the CD-29, CD-44, CD-73, and CD-90 which are markers expressing in mesenchymal stem cells were observed in both the STK and STK H, and the expression amounts were not different between the STK and STK H (see 105 through 112). Although not shown in Fig. 5, CD-11b, CD-34, and CD-45 which are markers that do not express in human mesenchymal stem cells (i.e., negative markers) were not seen in both the STK and STK H.

### <Conclusion of Example 3>

In the human bone marrow-derived mesenchymal stem cells cultured for 24 hours under the 21%O₂ environment with use of the STK2 medium and in the human bone marrow-derived mesenchymal stem cells cultured for 24 hours under the 1%O₂ environment with use of the STK2 medium, the specific surface antigens that express in human bone marrow-derived mesenchymal stem cells did not change. From this, it has been suggested that no influence is exerted on immunological tolerance of the human bone marrow-derived mesenchymal stem cells cultured for 24 hours under the 1%O₂ environment and on safety in implantation of the human bone marrow-derived mesenchymal stem cells.

### [Example 4]

Expression amounts of VEGF and HGF contained in culture supernatant of human bone marrow-derived mesenchymal stem cells cultured in a serum-free medium were studied.

### <Preparation of culture supernatant (conditioned medium)>

Human bone marrow-derived mesenchymal stem cells, which had been subcultured three or more times, were seeded to a 6-well plate, and cultured to be sub-confluent, with use of an STK2 medium. After the culture, the culture medium was changed from the STK2 medium to a serum-free DMEM, and culture was carried out for 24 hours under a 5% oxygen environment. After the 24-hour culture under the 5% oxygen environment, culture supernatant of the human bone marrow-derived mesenchymal stem cells was obtained as "5%O₂".

Human bone marrow-derived mesenchymal stem cells, which had been subcultured three or more times, were seeded to a 6-well plate, and cultured to be sub-confluent, with use of an STK2 medium. After the culture, the culture medium was changed from the STK2 medium to a serum-free DMEM, and culture was carried out for 24 hours under a 3% oxygen environment. After the 24-hour culture under the 3% oxygen environment, culture supernatant of the human bone marrow-derived mesenchymal stem cells was obtained as "3%O₂".

Human bone marrow-derived mesenchymal stem cells, which had been subcultured three or more times, were seeded to a 6-well plate, and cultured to be sub-confluent, with use of an STK2 medium. After the culture, the culture medium was changed from the STK2 medium to a serum-free DMEM, and culture was carried out for 24 hours under a 1% oxygen environment. After the 24-hour culture under the 1% oxygen environment, culture supernatant of the human bone marrow-derived mesenchymal stem cells was obtained as "1%O₂".

As a control, human bone marrow-derived mesenchymal stem cells, which had been subcultured three or more times, were seeded to a 6-well plate, and cultured to be sub-confluent, with use of an STK2 medium. After the culture, the culture medium was changed from the STK2 medium to a serum-free DMEM, and culture was carried out for 24 hours under a 21% oxygen environment. After the 24-hour culture under the 21% oxygen environment, culture supernatant of the human bone marrow-derived mesenchymal stem cells was obtained as "control".

A gas, which had been prepared to contain oxygen (1% or 21%), carbon dioxide (5%), and nitrogen (94% or 74%) and accommodated in a gas bottle, was supplied from the gas bottle to the 1% oxygen environment and to the 21% oxygen environment among the culture environments in which the human bone marrow-derived mesenchymal stem cells were cultured. Meanwhile, to the 3% oxygen environment and to the 5% oxygen environment, a gas obtained by mixing the gas supplied to the 1% oxygen environment with the gas supplied to the 21% oxygen environment at an appropriate ratio was supplied.

### <Evaluation Methods>

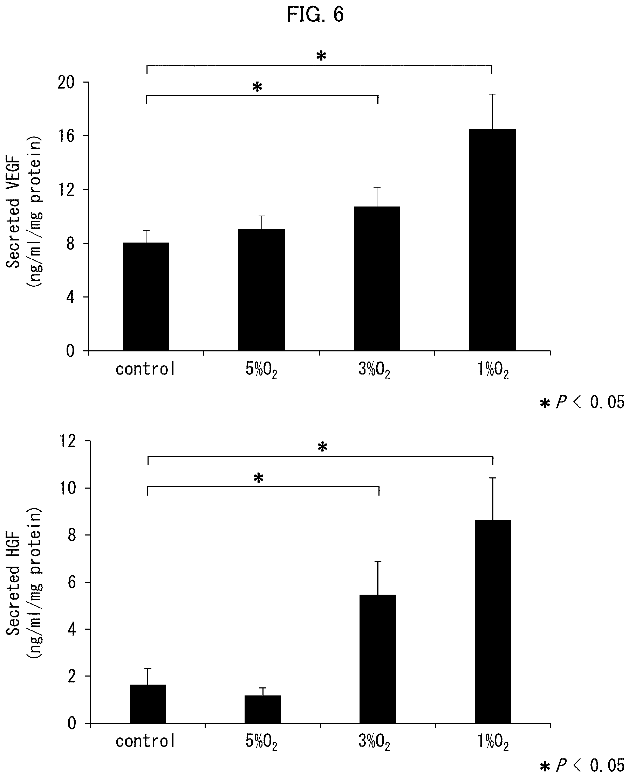

With an ELISA kit (R&D Systems, Minneapolis, Mn, USA) for measuring VEGF concentration, VEGF concentrations in the collected culture supernatants (i.e., 5%O₂, 3%O₂, 1%O₂, and control) were measured in n=6 for each group. Average values of the VEGF concentrations are shown in the upper part of Fig. 6. Note that each of the measured values was corrected for the entire protein amount in the culture supernatant, and a significance test was carried out by a Mann-Whitney U test to determine a significant difference from the control.

Moreover, with an ELISA kit (R&D Systems, Minneapolis, Mn, USA) for measuring an HGF concentration, HGF concentrations in the collected culture supernatants (i.e., 5%O₂, 3%O₂, 1%O₂, and control) were measured in n=6 for each group. Average values of the HGF concentrations are shown in the lower part of Fig. 6. Note that each of the measured values was corrected for the entire protein amount in the culture supernatant, and a significance test was carried out by a Mann-Whitney U test to determine a significant difference from the control.

### <Results>

As shown in the upper part in Fig. 6, in the 3%O₂, a significant increase in VEGF was found, as compared with the control. In the 1%O₂, an increase in VEGF greater than that in the 3%O₂ was found, as compared with the control.

As shown in the lower part in Fig. 6, in the 3%O₂, a significant increase in HGF was found, as compared with the control. In the 1%O₂, an increase in HGF greater than that in the 3%O₂ was found, as compared with the control.

### <Conclusion of Example 4>

In the human bone marrow-derived mesenchymal stem cells, the oxygen concentration needed to heighten the VEGF secretion property and HGF secretion property was less than 5%, preferably 3% or less, most preferably 1% or less.

### [Example 5]

Change with time of an amount of VEGF contained in culture supernatant of human bone marrow-derived mesenchymal stem cells cultured in a serum-free medium under a 1% oxygen environment was studied.

### <Preparation of culture supernatant>

Human bone marrow-derived mesenchymal stem cells, which had been subcultured three or more times, were seeded to a 6-well plate, and cultured to be sub-confluent, with use of an STK2 medium. After the culture, the culture medium was changed from the STK2 medium to a serum-free DMEM, and culture was carried out for 18 hours under a 21% oxygen environment, and then culture was carried out for 6 hours under a 1% oxygen environment. After the 6-hour culture under the 1% oxygen environment, culture supernatant of the human bone marrow-derived mesenchymal stem cells was obtained as "1%O₂ 6hr".

Human bone marrow-derived mesenchymal stem cells, which had been subcultured three or more times, were seeded to a 6-well plate, and cultured to be sub-confluent, with use of an STK2 medium. After the culture, the culture medium was changed from the STK2 medium to a serum-free DMEM, and culture was carried out for 12 hours under a 21% oxygen environment, and then culture was carried out for 12 hours under a 1% oxygen environment. After the 12-hour culture under the 1% oxygen environment, culture supernatant of the human bone marrow-derived mesenchymal stem cells was obtained as "1%O₂ 12hr".

Human bone marrow-derived mesenchymal stem cells, which had been subcultured three or more times, were seeded to a 6-well plate, and cultured to be sub-confluent, with use of an STK2 medium. After the culture, the culture medium was changed from the STK2 medium to a serum-free DMEM, and culture was carried out for 24 hours under a 1% oxygen environment. After the 24-hour culture under the 1% oxygen environment, culture supernatant of the human mesenchymal stem cells was obtained as "1%O₂ 24hr".

As a control, human bone marrow-derived mesenchymal stem cells, which had been subcultured three or more times, were seeded to a 6-well plate, and cultured to be sub-confluent, with use of an STK2 medium. After the culture, the culture medium was changed from the STK2 medium to a serum-free DMEM, and culture was carried out for 24 hours under a 21% oxygen environment. After the 24-hour culture under the 21% oxygen environment, culture supernatant of the human bone marrow-derived mesenchymal stem cells was obtained as "control".

A gas, which had been prepared to contain oxygen (1% or 21%), carbon dioxide (5%), and nitrogen (94% or 74%) and accommodated in a gas bottle, was supplied from the gas bottle to the environment in which the human bone marrow-derived mesenchymal stem cells were cultured.

### <Evaluation Methods>

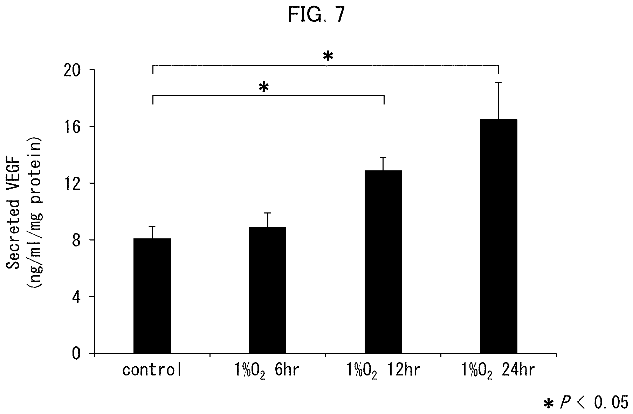

With an ELISA kit (R&D Systems, Minneapolis, Mn, USA) for measuring VEGF concentration, VEGF concentrations in the collected culture supernatants (i.e., 1%O₂ 6hr, 1%O₂ 12hr, 1%O₂ 24hr, and control) were measured in n=6 for each group. Average values of the VEGF concentrations are shown in Fig. 7. Note that each of the measured values was corrected for the entire protein amount in the culture supernatant, and a significance test was carried out by a Mann-Whitney U test to determine a significant difference from the control.

### <Results>

As shown in Fig. 7, in the 1%O₂ 12hr, a significant increase in VEGF was found, as compared with the control. In the 1%O₂ 24hr, an increase in VEGF greater than that in the 1%O₂ 12hr was found, as compared with the control.

### <Conclusion of Example 5>

It has been found that, in order to heighten the VEGF secretion property of the human bone marrow-derived mesenchymal stem cells under the low oxygen condition, the culture time is preferably 12 hours or more, further preferably 24 hours or more.

### [Example 6]

### <Culture of cell and collection of RNA>

(i) Human bone marrow-derived mesenchymal stem cells were subcultured three or more times with use of a DMEM medium containing 10% FBS. After that, the human bone marrow-derived mesenchymal stem cells were cultured for 24 hours under a 21% oxygen environment in a DMEM medium containing 10% FBS. Subsequently, according to a known method, mRNA was collected from the human bone marrow-derived mesenchymal stem cells. Hereinafter, the mRNA is referred to as "mRNA (1)".
(ii) Human bone marrow-derived mesenchymal stem cells were subcultured three or more times with use of an STK medium. After that, the human bone marrow-derived mesenchymal stem cells were cultured for 24 hours under a 1% oxygen environment in a serum-free medium. Subsequently, according to a known method, mRNA was collected from the human bone marrow-derived mesenchymal stem cells. Hereinafter, the mRNA is referred to as "mRNA (2)".
(iii) Human bone marrow-derived mesenchymal stem cells were subcultured three or more times with use of an STK medium. After that, the human bone marrow-derived mesenchymal stem cells were cultured for 24 hours under a 21% oxygen environment in a serum-free medium. Subsequently, according to a known method, mRNA was collected from the human bone marrow-derived mesenchymal stem cells. Hereinafter, the mRNA is referred to as "mRNA (3)".
(iv) Human bone marrow-derived mesenchymal stem cells were subcultured three or more times with use of a DMEM medium containing 10% FBS. After that, the human bone marrow-derived mesenchymal stem cells were cultured for 24 hours under a 1% oxygen environment in a DMEM medium containing 10% FBS. Subsequently, according to a known method, mRNA was collected from the human bone marrow-derived mesenchymal stem cells. Hereinafter, the mRNA is referred to as "mRNA (4)".

A gas, which contained oxygen (1% or 21%), carbon dioxide (5%), and nitrogen (94% or 74%), was supplied to the environment in which the human bone marrow-derived mesenchymal stem cells were cultured, and thus the oxygen concentration in the environment was adjusted.

### <Evaluation by microarray>

With a DNA microarray (Agilent Gene Expression Microarray: SurePrint G3 Human Gene Expression), amounts of mRNA of specific genes A (e.g., ANGPTL4, FABP3, DLK2, ALDOC, TCAF2, RCOR2, and the like) contained in each of the collected mRNAs (1) through (4) were measured.

Next, for each type of the genes, values of "mRNA amount of gene A contained in mRNA (2)/mRNA amount of gene A contained in mRNA (1)", "mRNA amount of gene A contained in mRNA (3)/mRNA amount of gene A contained in mRNA (1)", and "mRNA amount of gene A contained in mRNA (4)/mRNA amount of gene A contained in mRNA (1)" were calculated.

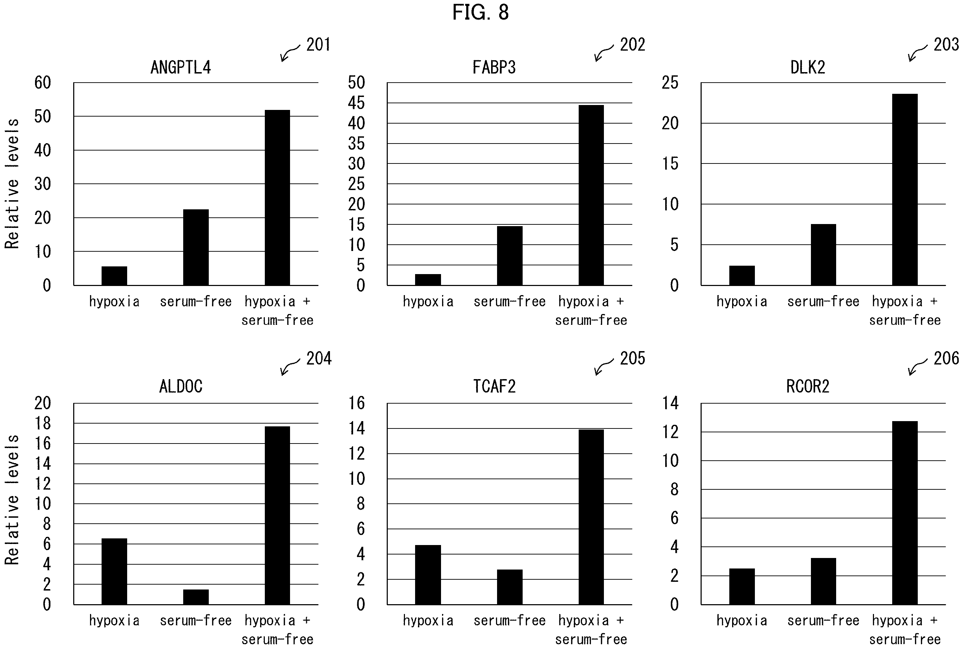

In Fig. 8 (described later), the value of "mRNA amount of gene A contained in mRNA (2)/mRNA amount of gene A contained in mRNA (1)" is indicated as "hypoxia+serum-free", the value of "mRNA amount of gene A contained in mRNA (3)/mRNA amount of gene A contained in mRNA (1)" is indicated as "serum-free", and the value of "mRNA amount of gene A contained in mRNA (4)/mRNA amount of gene A contained in mRNA (1)" is indicated as "hypoxia".

### <Results>

The graph 201 in Fig. 8 indicates comparison of expression amounts of ANGPTL4. The graph 202 in Fig. 8 indicates comparison of expression amounts of FABP3. The graph 203 in Fig. 8 indicates comparison of expression amounts of DLK2. The graph 204 in Fig. 8 indicates comparison of expression amounts of ALDOC. The graph 205 in Fig. 8 indicates comparison of expression amounts of TCAF2. The graph 206 in Fig. 8 indicates comparison of expression amounts of RCOR2.

As shown in the graph 201 in Fig. 8, the expression amount of ANGPTL4 in the hypoxia+serum-free was approximately 52 times, the expression amount of ANGPTL4 in the serum-free was approximately 22 times, and the expression amount of ANGPTL4 in the hypoxia was approximately 6 times.

As shown in the graph 202 in Fig. 8, the expression amount of FABP3 in the hypoxia+serum-free was approximately 45 times, the expression amount of FABP3 in the serum-free was approximately 15 times, and the expression amount of FABP3 in the hypoxia was approximately 3 times.

As shown in the graph 203 in Fig. 8, the expression amount of DLK2 in the hypoxia+serum-free was approximately 24 times, the expression amount of DLK2 in the serum-free was approximately 8 times, and the expression amount of DLK2 in the hypoxia was approximately twice.

As shown in the graph 204 in Fig. 8, the expression amount of ALDOC in the hypoxia+serum-free was approximately 18 times, the expression amount of ALDOC in the serum-free was approximately twice, and the expression amount of ALDOC in the hypoxia was approximately 7 times.

As shown in the graph 205 in Fig. 8, the expression amount of TCAF2 in the hypoxia+serum-free was approximately 14 times, the expression amount of TCAF2 in the serum-free was approximately 3 times, and the expression amount of TCAF2 in the hypoxia was approximately 5 times.

As shown in the graph 206 in Fig. 8, the expression amount of RCOR2 in the hypoxia+serum-free was approximately 13 times, the expression amount of RCOR2 in the serum-free was approximately 3 times, and the expression amount of RCOR2 in the hypoxia was approximately 3 times.

### <Conclusion of Example 6>

The expression amount of the gene A in mRNA collected from the human bone marrow-derived mesenchymal stem cells cultured for 24 hours under the 1% oxygen environment in the serum-free medium was, for all types, 3 or more times, as compared with the expression amount of the gene A in mRNA collected from the human bone marrow-derived mesenchymal stem cells cultured for 24 hours under the 21% oxygen environment in the DMEM medium containing 10% FBS.

The expression amount of DLK2 in mRNA collected from the human bone marrow-derived mesenchymal stem cells cultured for 24 hours under the 1% oxygen environment in the DMEM medium containing 10% FBS was approximately twice the expression amount of DLK2 in mRNA collected from the human bone marrow-derived mesenchymal stem cells cultured for 24 hours under the 21% oxygen environment in the DMEM medium containing 10% FBS. The expression amount of the gene A other than DLK2 in mRNA collected from the human bone marrow-derived mesenchymal stem cells cultured for 24 hours under the 1% oxygen environment in the DMEM medium containing 10% FBS was 3 or more times the expression amount of the gene A other than DLK2 in mRNA collected from the human bone marrow-derived mesenchymal stem cells cultured for 24 hours under the 21% oxygen environment in the DMEM medium containing 10% FBS.

The expression amount of ALDOC in mRNA collected from the human bone marrow-derived mesenchymal stem cells cultured for 24 hours under the 21% oxygen environment in the serum-free medium was approximately twice the expression amount of ALDOC in mRNA collected from the human bone marrow-derived mesenchymal stem cells cultured for 24 hours under the 21% oxygen environment in the DMEM medium containing 10% FBS. The expression amount of the gene A other than ALDOC in mRNA collected from the human bone marrow-derived mesenchymal stem cells cultured for 24 hours under the 21% oxygen environment in the serum-free medium was 3 or more times the expression amount of DLK2 in mRNA collected from the human bone marrow-derived mesenchymal stem cells cultured for 24 hours under the 21% oxygen environment in the DMEM medium containing 10% FBS.

For each type of the genes A, the mRNA amount of the gene A contained in the human bone marrow-derived mesenchymal stem cells cultured for 24 hours under the 1% oxygen environment in the serum-free medium was at least twice or more the mRNA amount of the gene A contained in the human bone marrow-derived mesenchymal stem cells cultured for 24 hours under the 21% oxygen environment in the serum-free medium. For each type of the genes A, the mRNA amount of the gene A contained in the human bone marrow-derived mesenchymal stem cells cultured for 24 hours under the 1% oxygen environment in the serum-free medium was at least twice or more the mRNA amount of the gene A contained in the human bone marrow-derived mesenchymal stem cells cultured for 24 hours under the 1% oxygen environment in the DMEM medium containing 10% FBS. That is, by culturing the human bone marrow-derived mesenchymal stem cells for 24 hours under the 1% oxygen environment in the serum-free medium, the expression amount of the gene A is greatly increased, as compared with the case of culture for 24 hours under the 1% oxygen environment in the DMEM medium containing 10% FBS and the case of culture for 24 hours under the 21% oxygen environment in the serum-free medium.

The mRNA amount of the gene A contained in the human bone marrow-derived mesenchymal stem cells cultured for 24 hours under the 1% oxygen environment in the serum-free medium was greater than the total of the mRNA amount of the gene A contained in the human bone marrow-derived mesenchymal stem cells cultured for 24 hours under the 21% oxygen environment in the serum-free medium and the mRNA amount of the gene A contained in the human bone marrow-derived mesenchymal stem cells cultured for 24 hours under the 1% oxygen environment in the DMEM medium containing 10% FBS. Thus, the synergistic effect was exhibited by the culture under the 1% oxygen environment and the culture in the serum-free medium.

### [Example 7]

### <Preparation of MSC>

Human bone marrow-derived mesenchymal stem cells which had been subcultured three or more times with use of an STK2 medium were cultured for 24 hours under a 1% oxygen environment in an STK2 medium. The human bone marrow-derived mesenchymal stem cells are referred to as "STK H". Moreover, human bone marrow-derived mesenchymal stem cells which had been subcultured three or more times with use of an STK2 medium were cultured for 24 hours under a 21% oxygen environment in an STK2 medium. The human bone marrow-derived mesenchymal stem cells are referred to as "STK".

Human bone marrow-derived mesenchymal stem cells which had been subcultured three or more times with use of a 10% bovine serum-containing DMEM medium were cultured for 24 hours under a 1% oxygen environment in a 10% bovine serum-containing DMEM medium. The human bone marrow-derived mesenchymal stem cells are referred to as "10%FBS H". Moreover, human bone marrow-derived mesenchymal stem cells which had been subcultured three or more times with use of a 10% bovine serum-containing DMEM medium were cultured for 24 hours under a 21% oxygen environment in a 10% bovine serum-containing DMEM medium. The human bone marrow-derived mesenchymal stem cells are referred to as "10%FBS".

### <Evaluation Methods>

To a 96-well dish, 100 pL/well of the STK2 medium or the 10% bovine serum-containing DMEM medium was added. To the STK2 medium, 2500 cells/well of the STK H or 2500 cells/well of the STK were seeded. To the 10% bovine serum-containing DMEM medium, 2500 cells/well of the 10%FBS H or 2500 cells/well of the 10%FBS were seeded.

The STK H and 10%FBS H were continued to be cultured under the 1% oxygen environment, and the STK and 10%FBS were continued to be cultured under the 21% oxygen environment. To each of the wells, 10 pL/well of Premix WST-1 (Takara bio) was added each time of 0 hours later, 12 hours later, and 24 hours later. After 4 hours had elapsed from the addition, absorbance was measured at a wavelength of 450 nm, and the number of cells was quantitatively determined based on the measured value (n=5 for each group).

### <Results>

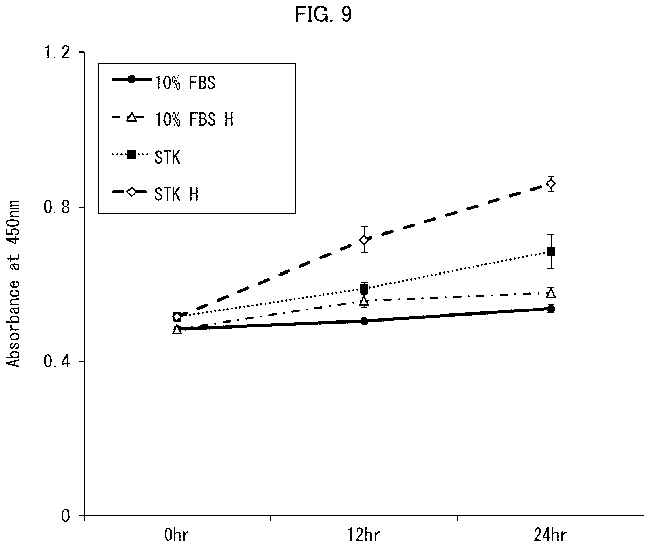

As shown in Fig. 9, the 10%FBS H, STK, STK H exhibited the significantly large number of cells at all the points in time (i.e., 0 hours, 12 hours, 24 hours) as compared with the 10%FBS, and it has been thus indicated that cell growth was enhanced. The STK H had the highest cell growth capacity, and showed the significantly greater number of cells at all the points in time, as compared with the STK.

### <Conclusion of Example 7>

It has been indicated that, by the culture in the serum-free medium, the capacity of growing human bone marrow-derived mesenchymal stem cells was enhanced under the 1% oxygen environment and in the 21% oxygen environment, as compared with the 10% bovine serum-containing DMEM medium. Further, it has been indicated that the cell growth capacity was mostly enhanced for the human bone marrow-derived mesenchymal stem cells cultured under the 1% oxygen environment with use of the serum-free medium.

### [Example 8]

Expression amounts of VEGF and HGF contained in culture supernatant of human bone marrow-derived mesenchymal stem cells subcultured in an FBS-containing DMEM medium or in STK2 (i.e., serum-free medium) were studied.

### <Preparation of culture supernatant (conditioned medium) and evaluation method>

Human bone marrow-derived mesenchymal stem cells, which had been subcultured three or more times, were seeded to a 6-well plate, and cultured to be sub-confluent, with use of a 10% FBS-containing DMEM medium. After the culture, the culture medium was changed from the 10% FBS-containing DMEM medium to a serum-free DMEM medium, and culture was carried out for 24 hours under a 1% oxygen environment or a 21% oxygen environment. After the 24-hour culture under the 1% oxygen environment, culture supernatant of the human bone marrow-derived mesenchymal stem cells was obtained as "DMEM 1%O₂". Moreover, after the 24-hour culture under the 21% oxygen environment, culture supernatant of the human bone marrow-derived mesenchymal stem cells was obtained as "DMEM 21%O₂".

Moreover, human bone marrow-derived mesenchymal stem cells, which had been subcultured three or more times, were seeded to a 6-well plate, and cultured to be sub-confluent, with use of an STK2 medium. After the culture, the culture medium was changed from the STK2 medium to a serum-free DMEM medium, and culture was carried out for 24 hours under a 1% oxygen environment or a 21% oxygen environment. After the 24-hour culture under the 1% oxygen environment, culture supernatant of the human bone marrow-derived mesenchymal stem cells was obtained as "STK2 1%O₂". Moreover, after the 24-hour culture under the 21% oxygen environment, culture supernatant of the human bone marrow-derived mesenchymal stem cells was obtained as "STK2 21%O₂".

As a control, human renal proximal tubular epithelial cells (HK2 cells) were cultured for 24 hours under a 21% oxygen environment with use of a serum-free DMEM medium. After the 24-hour culture under the 21% oxygen environment, culture supernatant of the human kidney cells was obtained as "HK2".

A gas, which had been prepared to contain oxygen (1% or 21%), carbon dioxide (5%), and nitrogen (94% or 74%) and accommodated in a gas bottle, was supplied from the gas bottle to the 1% oxygen environment and the 21% oxygen environment among the culture environments in which the human bone marrow-derived mesenchymal stem cells were cultured.

For each of the culture supernatants, the VEGF concentration and the HGF concentration were measured by a method similar to that of Example 4.

### <Results>

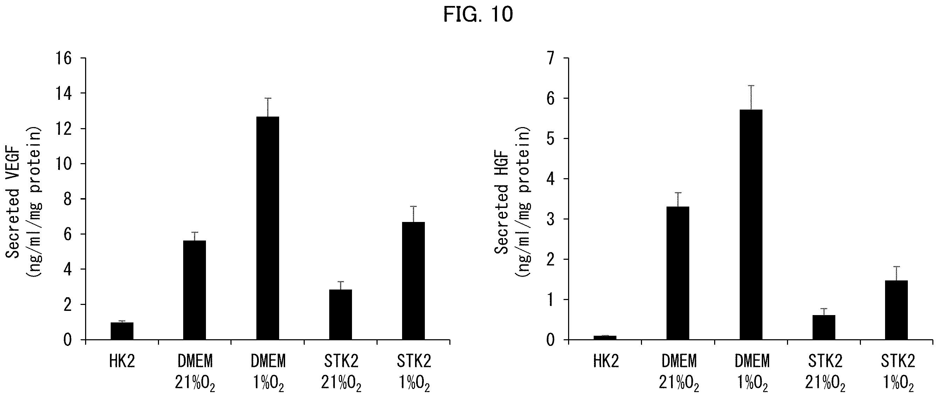

As shown in Fig. 10, it has been indicated that the VEGF expression amount and the HGF expression amount were decreased in the case of subculture in the STK2 medium, as compared with the case of subculture in the 10% FBS-containing DMEM medium. Meanwhile, it has been found that, in both the cases of subculture in the 10% FBS-containing DMEM medium and of subculture in the STK2 medium, the VEGF expression amount and the HGF expression amount were increased under the 1% oxygen, as compared with the case of culture under the 21% oxygen. Moreover, it has been found that the VEGF expression amount and the HGF expression amount in the case of subculture in the STK2 medium were extremely greater than the VEGF expression amount and the HGF expression amount in the HK2.

### <Conclusion of Example 8>

It has been indicated that the VEGF secretion property and HGF secretion property of the human bone marrow-derived mesenchymal stem cells are greatly higher, as compared with the human proximal tubular epithelial cells. Moreover, it has been indicated that, in the case where the human bone marrow-derived mesenchymal stem cells are subcultured in the serum-free medium, the VEGF expression amount and the HGF expression amount are recovered by the culture under the 1% oxygen, although the VEGF secretion property and HGF secretion property are lowered.

### Industrial Applicability

The present invention and the repair agent prepared by the production method of the present invention are applicable to repair agents for damaged tissue.

## Claims

1. A method for producing a repair agent for damaged tissue, said method comprising the step of:
culturing mesenchymal stem cells in a serum-free medium at an oxygen concentration of less than 5%.

2. The method as set forth in claim 1, wherein the serum-free medium contains an FGF, a PDGF, an EGF, at least one phospholipid, and at least one fatty acid.

3. The method as set forth in claim 1 or 2, wherein the mesenchymal stem cells are subcultured at least once in the culturing step.

4. A repair agent for damaged tissue, said repair agent comprising mesenchymal stem cells in which a gene that encodes angiopoietin-like 4, a gene that encodes fatty acid binding protein 3, a gene that encodes delta-like 2 homolog (Drosophila), a gene that encodes fructose-bisphosphate aldolase C, a gene that encodes TRPM8 channel-associated factor 2, and a gene that encodes REST corepressor 2 are respectively expressed in amounts of at least 3 or more times, as compared with respective expression amounts thereof in mesenchymal stem cells which have been cultured in a serum-containing medium at an oxygen concentration of 5% or more.

5. The repair agent as set forth in claim 4, wherein said repair agent is intended to (i) suppress fibrosis of a living tissue, (ii) suppress infiltration of an inflammatory cell, or (iii) control activity of a macrophage.

6. The repair agent as set forth in claim 4 or 5, wherein:
the damaged tissue accompanies acute kidney injury, chronic kidney disease, chronic renal failure, chronic glomerulonephritis, diabetic nephropathy, nephrosclerosis, rapidly progressive glomerulonephritis, polycystic kidney, tubulointerstitial nephritis, drug-induced nephritis, lupus nephritis, hydronephrosis, gouty kidney, cirrhosis, pulmonary fibrosis, a burn, interstitial pneumonia, drug-induced pneumonia, irradiation pneumonitis, chronic obstructive pulmonary disease, an acute respiratory distress syndrome, cartilage damage, a bone defect, spinal cord damage, periodontal disease, multiple sclerosis, rheumatoid arthritis, systemic lupus erythematosus, Crohn's disease, diabetes, arteriosclerosis, myocardial infarction, stroke, Alzheimer's disease, macular degeneration, viral hepatitis, alcoholic hepatitis, non-alcoholic steatohepatitis, jawbone reconstruction, cleft palate, a bone replacement material, a bone defect, a bone system disease, a dry eye, a corneal disorder, pharyngitis, arthritis, a cancer, cancer neighborhood fibrosis, or fibrosis.
